# EUROPEAN PATENT APPLICATION

(11) **EP 4 305 987 A1**
(43) Date of publication of application: **17.01.2024**
(21) Application number: 23184165.1
(22) Date of filing: 07.07.2023
(51) Int. Cl.: A24F 40/53, A61M 16/00

(54) **RESIDUAL AMOUNT MEASUREMENT AEROSOL GENERATING DEVICE**

(30) Priority: 11.07.2022 KR 20220084858; 22.07.2022 KR 20220090853; 29.07.2022 KR 20220094635
(71) Applicant: Em-tech. Co., Ltd., Changwon-si, Gyeongsangnam-do 51539 (KR)
(72) Inventor: KWON, Joong Hak, 14038 Anyang-si, Gyeonggi-do (KR); KIM, Jun Hee, 48516 Nam-gu, Busan (KR)
(74) Representative: Müller Hoffmann & Partner

(57) **Abstract**

Embodiments relate to a residual amount measurement aerosol generating device, and more particularly, to an aerosol generating device capable of calculating a residual amount of an aerosol-forming substrate due to puff and calculating a residual number of puffs of a cigarette or appropriately controlling a temperature using the calculated residual amount of an aerosol-forming substrate.

The aerosol generating device of an embodiment includes a case including a heating space and an air flow path connecting the heating space to an external space, a heating unit heating an aerosol-forming article inserted into the heating space, a puff factor detecting unit detecting a factor related to a user's puff, and a controller controlling at least the heating unit and calculating a residual amount of the aerosol-forming substrate based on detection contents of the puff factor detecting unit.

## Description

### TECHNICAL FIELD

Embodiments relate to a residual amount measurement aerosol generating device, and more particularly, to an aerosol generating device capable of calculating a residual amount of an aerosol-forming substrate due to puff and calculating a residual number of puffs of a cigarette or appropriately controlling a temperature using the calculated residual amount of an aerosol-forming substrate.

### BACKGROUND ART

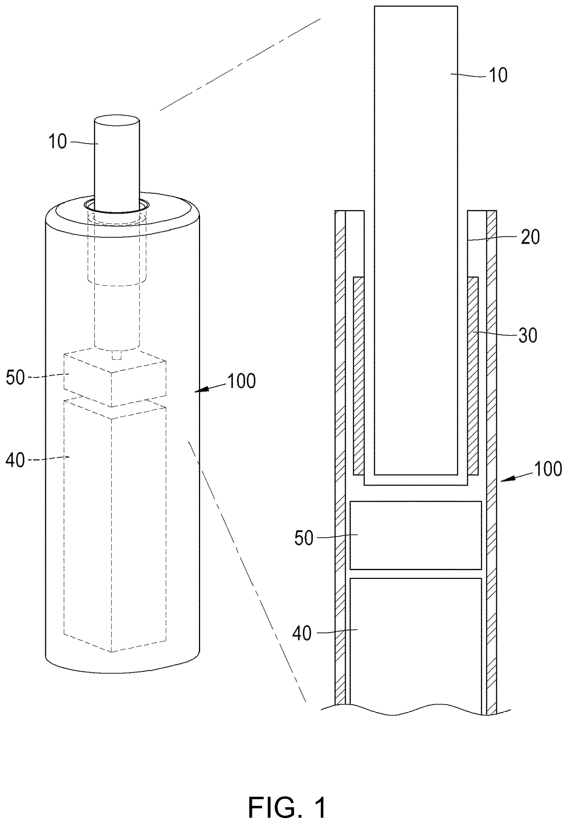

An aerosol is a suspension of fine solid particles or liquid droplets in air and usually has a size of about 0.001 to about 1.0 pm. In particular, there are cases in which a person inhales aerosols derived from various types of cigarette-type aerosol-forming articles. For example, according to demand of consumers who prefer general cigarette-type cigarettes, an electronic cigarette having a shape of a filter portion and a cigarette portion of general cigarettes has also been proposed. The electronic cigarette is configured such that, when an inhalation material included in the cigarette portion is vaporized by an electronic heater, a user inhales through the filter portion having the same configuration as that of the general cigarettes. FIG. 1 is a view illustrating an example of an aerosol generating device according to the related art. Referring to FIG. 1, an aerosol generating device 100 includes a cavity 20 into which a cigarette-type aerosol-forming article 10 is inserted and a pipe-shaped heater 30 provided in an outer circumference of the cavity 20 to heat the cigarette-type aerosol-forming article 10 inserted into the aerosol generating device 100 to generate an aerosol. The aerosol generating device 100 further includes a battery 40 for supplying electric power to the heater 30 and a controller 50 configured to control electric power supplied from the battery 40 to the heater 30. In the related art described above, electric power is supplied from the battery 40 to the heater 30 under the control of the controller 50, and the cigarette-type aerosol-forming article 10 is heated by heat generated by the heater 30 and an aerosol is generated from the aerosol-forming substrate within the cigarette-type aerosol-forming article 10.

Quality of an aerosol generating device depends on user experience, such as the degree of generating an aerosol, the taste and aroma that the user feels when inhaling, and the amount of mist. In particular, Korean Application Publication Nos. 10-2022-0074974 and 10-2019-0057399 disclose a method of measuring a residual amount of an aerosol-forming substrate by detecting a user's puff (inhalation) action or detecting the residual amount of liquid in a liquid cartridge. The residual number of puffs may be calculated through the detection of the puff action or the residual amount of liquid, and based on this, beneficial effects, such as control of the heater, notification of the residual number of times, and warning of the amount of nicotine intake may be provided and user experience may be improved. However, especially in the case of the cigarette-type aerosol-forming article, it may be difficult to accurately measure the residual amount of the aerosol-forming substrate, and thus, a method for improving this is required.

### DISCLOSURE OF THE INVENTION

An aspect of the present disclosure is to provide an aerosol generating device capable of measuring or estimating the consumption amount of an aerosol-forming substrate through puff-related factors, such as a change in pressure or a change in temperature when calculating the residual amount of an aerosol-forming substrate, thereby advantageously performing control.

According to an aspect of the present disclosure for achieving the above objects, there is provided an aerosol generating device including: a case including a heating space and an air flow path connecting the heating space to an external space; a heating unit heating an aerosol-forming article inserted into the heating space; a puff factor detecting unit detecting a factor related to a user's puff; and a controller controlling at least the heating unit and calculating a residual amount of the aerosol-forming substrate based on detection contents of the puff factor detecting unit, wherein the factor related to the user's puff detected by the puff factor detecting unit includes any one or more of a change in pressure in the air flow path, a flow rate of air flowing through the air flow path, a change in temperature in the heating space or the heating unit, or whether a user's puff is generated.

Also, in an aerogel generating device of an embodiment, the puff factor detecting unit may detect at least a change in pressure in the air flow path, and the controller may calculate the residual amount of the aerosol-forming substrate based on the change in pressure in the air flow path over time detected by the puff factor detecting unit.

Also, in an aerogel generating device of an embodiment, the controller may calculate the residual amount of the aerosol-forming substrate based on an integral value or an extreme value of the change in pressure in the air flow path over time detected by the puff factor detecting unit.

Also, in an aerogel generating device of an embodiment, the puff factor detecting unit may detect at least the flow rate of the air flowing through the air flow path, and the controller may calculate the residual amount of the aerosol-forming substrate based on the flow rate of the air flowing through the air flow path detected by the puff factor detecting unit.

Also, in an aerogel generating device of an embodiment, the puff factor detecting unit may detect at least whether a user's puff is generated, and the controller may calculate the residual amount of the aerosol-forming substrate based on whether the user's puff is generated, detected by the puff factor detecting unit.

Also, in an aerogel generating device of an embodiment, the controller may calculate the residual amount of the aerosol-forming substrate after the puff based on the residual amount of the aerosol-forming substrate before the puff.

Also, in an aerogel generating device of an embodiment, the controller may calculate the residual amount of the aerosol-forming substrate after the puff based on a temperature of the heating space or the heating unit before the puff.

Also, in an aerogel generating device of an embodiment, the controller may calculate the residual amount of the aerosol-forming substrate after the puff based on a duration of the puff.

Also, in an aerogel generating device of an embodiment, when calculating the residual amount of the aerosol-forming substrate, the controller may correct the residual amount of the aerosol-forming substrate by subtracting a consumption amount (a passive consumption amount) of the aerosol-forming substrate naturally consumed in a pause section between the user's puff actions.

Also, in an aerogel generating device of an embodiment, the controller may calculate the passive consumption amount by multiplying a reference consumption rate by a duration of the pause section.

Also, in an aerogel generating device of an embodiment, the reference consumption rate may be determined according to the residual amount of the aerosol-forming substrate.

Also, in an aerogel generating device of an embodiment, the reference consumption rate may be determined according to a temperature of the heating space or the heating unit.

Also, in an aerogel generating device of an embodiment, the reference consumption rate may be determined by an elapsed time of a current pause section.

Also, in an aerogel generating device of an embodiment, the puff factor detecting unit may detect at least a change in temperature of the heating space or the heating unit, and the controller may calculate the residual amount of the aerosol-forming substrate based on a change in temperature of the heating space or the heating unit over time detected by the puff factor detecting unit.

Also, in an aerogel generating device of an embodiment, the controller may calculate the residual amount of the aerosol-forming substrate based on an integral value or an extreme value of the change in temperature of the heating space or the heating unit over time detected by the puff factor detecting unit.

The aerosol generating device according to an embodiment may further include: a notification unit generating at least one of a haptic effect, a visual effect, or a sound effect for user notification, wherein the controller may calculate the number of possible puffs of the mounted aerosol-forming article based on the calculated residual amount of the aerosol-forming substrate, and informs the user of the calculated number of possible puffs through the notification unit.

Also, in an aerogel generating device of an embodiment, the controller may stop the heating of the heating unit when the flow rate of the air flowing through the air flow path detected by the puff factor detecting unit reaches a predetermined stop threshold value.

Also, in an aerogel generating device of an embodiment, when the operation of the aerosol generating device is stopped during use, the controller may store a residual amount of the aerosol-forming substrate calculated by then, and when the aerosol generating device is restarted, the controller may load the stored residual amount of the aerosol-forming substrate and continue to calculate the residual amount of the aerosol-forming substrate.

Also, in an aerogel generating device of an embodiment, the controller may initialize the calculated residual amount of the aerosol-forming substrate when the inserted aerosol-forming article is replaced.

The aerosol generating device according to an embodiment may further include: a temperature sensor detecting a temperature of the heating space or the heating unit and applying a temperature detection value to the controller, wherein the controller may control the heating unit according to a temperature profile including a target temperature and the temperature detection value to heat the aerosol-forming substrate of the aerosol-forming article to generate an aerosol, and adjust a target temperature of a next puff by reflecting the factor related to the user's puff detected by the puff factor detecting unit or the calculated residual amount of the aerosol-forming substrate.

Also, in an aerogel generating device of an embodiment, the controller may decrease a target temperature when the calculated residual amount of the aerosol-forming substrate is equal to or greater than a reference residual amount, and increase the target temperature when the calculated residual amount of the aerosol-forming substrate is less than the reference residual amount.

The aerosol generating device according to an embodiment may further include: a temperature sensor detecting a temperature of the heating space or the heating unit and applying a temperature detection value to the controller; and an input unit acquiring a variable input for a basic number of puffs from the user and applying the acquired variable input to the controller, wherein the controller increases a target temperature when the basic number of puffs is decreased, and decreases the target temperature when the basic number of puffs is increased.

According to embodiments, the consumption amount of an aerosol-forming substrate may be estimated using factors related to the user's puff, and the residual amount of the aerosol-forming substrate may be calculated using the estimated consumption amount of the aerosol-forming substrate.

In addition, according to the embodiments, the residual amount of the aerosol-forming substrate may be more accurately calculated by reflecting the passive consumption amount of the aerosol-forming substrate due to residual heat during the resting section between puffs.

In addition, the residual number of puffs may be accurately calculated, and based on this, the residual number of puffs or the residual amount of the aerosol-forming substrate may be displayed to the user.

In addition, the embodiment has an effect of maintaining a constant amount of mist or increasing the amount of mist by adjusting a target temperature to reflect factors related to the user's puff.

### BRIEF DESCRIPTION OF THE DRAWINGS

FIG. 1 is an internal configuration diagram illustrating an example of an aerosol generating device according to the related art;
FIG. 2 is a block diagram illustrating functional components of an aerosol generating device according to an embodiment of the present disclosure;
FIG. 3 is an internal configuration diagram conceptually illustrating a configuration of an aerosol generating device according to an embodiment of the present disclosure;
FIG. 4 is a table illustrating a relationship between a puff flow rate determined by an experiment and the consumption amount of an aerosol-forming substrate that may be used by a controller;
FIG. 5 is a graph illustrating a change in pressure over time detected by a puff factor detecting unit when the puff factor detecting unit includes a pressure sensor;
FIG. 6 is a graph illustrating a change in pressure over time detected by a pressure sensor of the puff factor detecting unit to explain another embodiment of the controller;
FIG. 7A is an example of a correspondence table for a controller to estimate the consumption amounts of an aerosol-forming substrate based on integrated values of changes in pressure over time by a controller, which may be determined by an experiment in advance, and FIG. 7B is an example of a correspondence table for a controller to estimate the consumption amounts of an aerosol-forming substrate based on peak values of changes in change in pressure over time;
FIG. 8 is a graph illustrating a change in temperature of a heating space or a heating unit over time detected by a puff factor detecting unit when the puff factor detecting unit includes a temperature sensor;
FIG. 9 is a graph of consumption of a residual amount of an aerosol-forming substrate residual amount consumption graph modeling how an aerosol-forming substrate in an aerosol-forming article is consumed in an aerosol generating device;
FIG. 10 is a graph illustrating the consumption amount of an aerosol-forming substrate or a flow rate of a puff during one puff, estimated by a controller, over time according to an embodiment of the present disclosure; and
FIG. 11 is a conceptual diagram illustrating how the residual amount of an aerosol-forming substrate calculated is displayed through a notification unit according to an embodiment of the present disclosure.

### BEST MODE FOR CARRYING OUT THE INVENTION

Hereinafter, various embodiments of the present disclosure will be described with reference to the accompanying drawings. Accordingly, those of ordinary skill in the art will recognize that modification, equivalent, and/or alternative on the various embodiments described herein may be variously made without departing from the scope and spirit of the present disclosure. With regard to description of drawings, similar components may be marked by similar reference numerals.

In the disclosure disclosed herein, the expressions "have," "may have," "include" and "comprise," or "may include" and "may comprise" used herein indicate existence of corresponding features (for example, elements, such as numeric values, functions, operations, or components) but do not exclude presence of additional features.

In the disclosure disclosed herein, the expressions "A or B," "at least one of A or/and B," or "one or more of A or/and B," and the like used herein may include any and all combinations of one or more of the associated listed items. For example, the term "A or B," "at least one of A and B," or "at least one of A or B" may refer to all of the case **(1)** where at least one A is included, the case **(2)** where at least one B is included, or the case **(3)** where both of at least one A and at least one B are included.

The terms, such as "first," "second," and the like used herein may refer to various elements of various embodiments of the present disclosure, but do not limit the elements. For example, such terms are used only to distinguish an element from another element and do not limit the order and/or priority of the elements. For example, a first user device and a second user device may represent different user devices irrespective of sequence or importance. For example, without departing the scope of the present disclosure, a first element may be referred to as a second element, and similarly, a second element may be referred to as a first element.

It will be understood that when an element (for example, a first element) is referred to as being "(operatively or communicatively) coupled with/to" or "connected to" another element (for example, a second element), it may be directly coupled with/to or connected to the other element or an intervening element (for example, a third element) may be present. In contrast, when an element (for example, a first element) is referred to as being "directly coupled with/to" or "directly connected to" another element (for example, a second element), it should be understood that there are no intervening element (for example, a third element).

According to the situation, the expression "configured to" used herein may be used as, for example, the expression "suitable for," "having the capacity to," "designed to," "adapted to," "made to," or "capable of." The term "configured to (or set to)" must not mean only "specifically designed to" in hardware. Instead, the expression "a device configured to" may mean that the device is "capable of" operating together with another device or other components. CPU, for example, a "processor configured to (or set to) perform A, B, and C" may mean a dedicated processor (for example, an embedded processor) for performing a corresponding operation or a generic-purpose processor (for example, a central processing unit (CPU) or an application processor) which may perform corresponding operations by executing one or more software programs which are stored in a memory device.

Terms used in this specification are used to describe specified embodiments of the present disclosure and are not intended to limit the scope of the present disclosure. The terms of a singular form may include plural forms unless otherwise specified. Unless otherwise defined herein, all the terms used herein, which include technical or scientific terms, may have the same meaning that is generally understood by a person skilled in the art. It will be further understood that terms, which are defined in a dictionary and commonly used, should also be interpreted as is customary in the relevant related art and not in an idealized or overly formal detect unless expressly so defined herein in various embodiments of the present disclosure. In some cases, even if terms are terms which are defined in the specification, they may not be interpreted to exclude embodiments of the present disclosure.

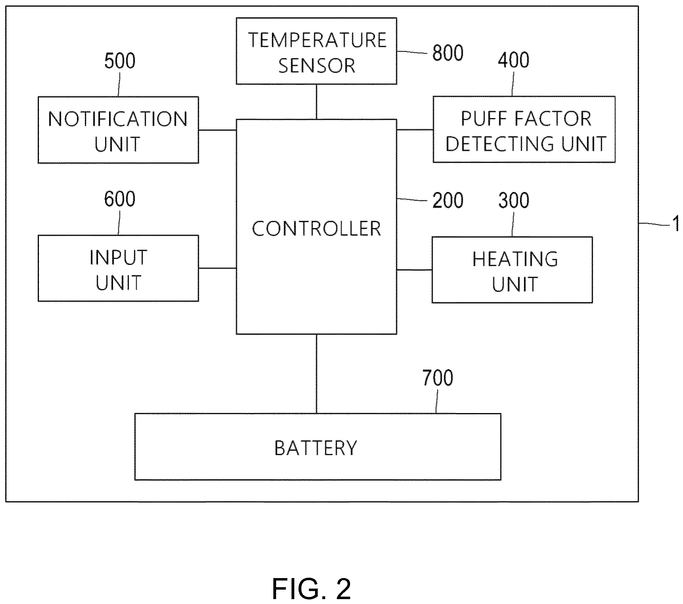

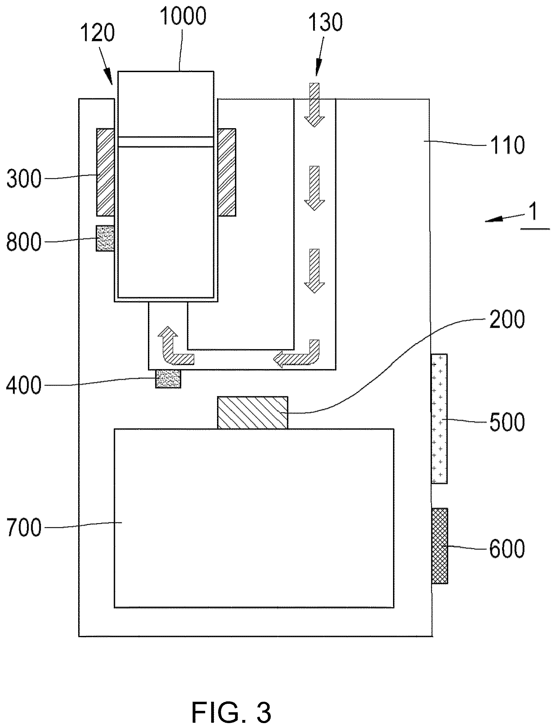

FIG. 2 is a block diagram illustrating functional components of an aerosol generating device 1 according to an embodiment of the present disclosure, and FIG. 3 is an internal configuration diagram conceptually illustrating a configuration of the aerosol generating device according to an embodiment of the present disclosure. In the present embodiment, the aerosol generating device 1 heats a cigarette-type aerosol-forming article 1000 inserted into a predetermined heating space 120 therein and converts an aerosol-forming substrate included therein into an aerosol. At this time, the user may inhale the generated aerosol through a puff (inhalation) action. In other embodiments, the aerosol generating device may accommodate an aerosol-forming article in the form of a liquid cartridge and heat a liquid aerosol-forming substrate stored therein, but all embodiments are not limited to the aerosol generating device accommodating the cigarette-type aerosol-forming article 1000.

The aerosol generating device 1 may include, for example, a controller 200 that controls the overall device, a heating unit 300 that heats the aerosol-forming article 1000, an a puff factor detecting unit 400 that detects factors related to the user's puff, a notification unit 500 and an input unit 600 that interact with a user, a battery 700 that applies electric power to components, and a temperature sensor 800 that detects a temperature of the heating space 120 or the heating unit 300 and applies a temperature detection value to the controller 200. In addition, the aerosol generating device 1 may include a case 110 that protects internal components, the heating space 120 defined in the case 110 and allowing the aerosol-forming article 1000 to be inserted thereinto, and an air flow path 130 that connects the heating space 120 to an external space.

The heating unit 300, substantially a heating element, may have various materials and structures and may generate an aerosol by applying heat to the aerosol-forming article 1000 inserted into the heating space 120. For example, the heating unit 300 may be a surface heating element having a heating pattern formed thereon. Alternatively, the heating unit 300 may be a susceptor that is inductively heated by a nearby electromagnetic induction coil. Heating of the heating unit 300 is controlled by the controller 200.

The puff factor detecting unit 400 detects a change in a measurable factor in the aerosol generating device 1 generated by a user's puff action. For example, the factor related to the user's puff detected by the puff factor detecting unit 400 may be a change in pressure in the air flow path 130. In this case, the puff factor detecting unit 400 may include, for example, a pressure sensor capable of detecting pressure in the air flow path 130. Also, in this case, the puff factor detecting unit 400 may measure the change in pressure over time in the air flow path 130 generated by the user's one-time puff action, estimates a puff flow rate per puff or a consumption amount of the aerosol-forming substrate included in the aerosol-forming article 1000 per puff, and calculate a residual amount of the aerosol-forming substrate based on the estimated puff flow rate or the estimated consumption amount of the aerosol-forming substrate.

Alternatively, for example, the factor related to the user's puff detected by the puff factor detecting unit 400 may be a flow rate of an air current flowing through the air flow path 130. In this case, the puff factor detecting unit 400 may include, for example, a flow sensor that directly measures a flow rate of the air current flowing through the air flow path 130. Also, in this case, the puff factor detecting unit 400 may directly measure a flow rate (a puff flow rate) of the air flowing through the air flow path 130 generated by the user's one-time puff action, and through this, the controller 200 may estimate a consumption amount of the aerosol-forming substrate included in the article 1000 per puff and calculate a residual amount of the aerosol-forming substrate based on the estimated residual amount.

Alternatively, for example, the factor related to the user's puff detected by the puff factor detecting unit 400 may be a change in temperature of the heating space 120 or the heating unit 300. In this case, the puff factor detecting unit 400 may include, for example, a temperature sensor installed in the heating space 120 or near the heating unit 300. Also, in this case, the puff factor detecting unit 400 may measure a variation of temperature over time in the air flow path 130 generated by the user's one-time puff action, and through this, the controller 200 may estimate a puff flow rate per puff or a consumption amount of the aerosol-forming substrate included in the aerosol-forming article 1000 per puff, and calculate a residual amount of the aerosol-forming substrate may be calculated based on the estimated puff flow rate or the estimated consumption amount of the aerosol-forming substrate.

Alternatively, for example, the factor related to the user's puff detected by the puff factor detecting unit 400 may be whether the user's puff is generated. In this case, the puff factor detecting unit 400 may include, for example, any one of the pressure sensor, the flow sensor, or the temperature sensor described above. At this time, for example, the puff factor detecting unit 400 may detect that the user's puff action takes place when pressure inside the air flow path is temporarily lowered due to the user's puff action, may detect that the user's puff action takes place when a flow rate of the air flowing through the air flow path 130 temporarily increases, and may detect that the user's puff action takes place when the temperature of the heating space 120 or the heating unit 300 temporarily decreases.

According to the above embodiment, an installation position of the puff factor detecting unit 400 may be appropriately selected. For example, when the puff factor detecting unit 400 is a pressure sensor or a flow sensor for measuring pressure or a flow rate in the air flow path 130, the pressure sensor or the flow sensor may be located in or nearby the air flow path 130. In addition, when the puff factor detecting unit 400 is a temperature sensor that measures the temperature of the heating space 120 or the heating unit 300, the puff factor detecting unit 400 may be located in the heating space 120 or near the heating unit 300. The factor related to the user's puff detected by the puff factor detecting unit 400 may be not one but several factors, and the puff factor detecting unit 400 may include several sensors, instead of a single sensor, corresponding thereto.

The controller 200 may include, for example, a micro-controller unit (MCU) capable of performing command processing, various operations, and device control. In addition, the controller 200 may include an electric power circuit or a switching element for controlling electric power applied from the battery 700 to the heating unit 300.

The controller 200 may perform overall control of the aerosol generating device 1, such as controlling the operation of the heating unit 300 and interacting with the user through the notification unit 500 and the input unit 600. In addition, the controller 200 calculates a residual amount of the aerosol-forming substrate in the currently inserted aerosol-forming article 1000 based on the detected contents of the puff factor detecting unit 400. At this time, a method of calculating the residual amount performed by the controller 200 may vary according to the configuration of the sensor included in the puff factor detecting unit 400. In addition, according to an embodiment, the controller 200 may perform an aerosol generating function of adjusting a target temperature of a next puff or puffs by reflecting factors related to the user's puff or the residual amount of the aerosol-forming substrate and controlling the heating unit 300 according to the adjusted target temperature, which is described in detail below.

As an example of the method of calculating a residual amount performed by the controller 200, the controller 200 may calculate a consumption amount of the aerosol-forming substrate through the puff factor detecting unit 400 and subtracting the calculated consumption amount from a previous residual amount to calculate a current residual amount. For example, when the puff factor detecting unit 400 includes a flow sensor that directly measures a flow rate of air flowing through the air flow path 130, the puff factor detecting unit 400 may directly detect the puff flow rate, and thus, the puff factor detecting unit 400 may apply the detection contents to the controller 200, and controller 200 may calculate the residual amount of the aerosol-forming substrate based thereon.

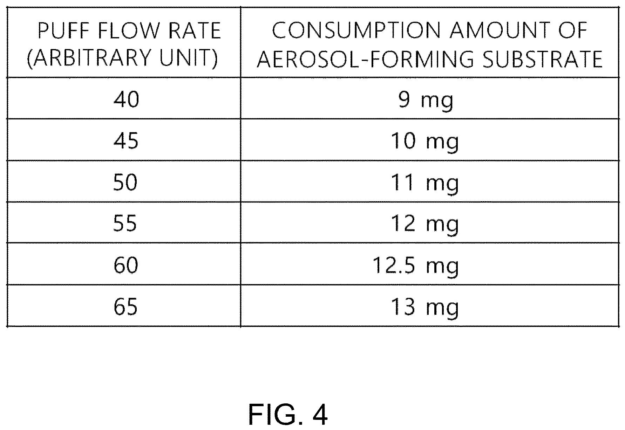

To this end, first, it is necessary to derive a relationship between the detected puff flow rate and the consumption amount of the aerosol-forming substrate in advance by an experiment and perform modeling or approximate modeling. The simplest modeling of the puff flow rate and the consumption amount of the aerosol-forming substrate is a linear proportional relationship. Alternatively, as shown in the table of the relationship between the puff flow rate and the aerosol-forming substrate consumption amount in FIG. 4, the puff flow rate measured through the puff factor detecting unit 400 and the average consumption amount of the actual aerosol-forming substrate may be derived through experiment, and a correspondence relationship may be made into a table and stored in the controller 200. At this time, the controller 200 may calculate a consumption amount of the aerosol-forming substrate for one puff based on the correspondence relationship in the table and use the calculated consumption amount to calculate a residual amount.

When the puff factor detecting unit 400 includes a pressure sensor capable of detecting pressure in the air flow path 130, the controller 200 may estimate the puff flow rate based on a change in pressure over time detected by the puff factor detecting unit 400 and calculate a consumption amount of the aerosol-forming substrate for one puff therethrough to use the calculated consumption amount to calculate the residual amount.

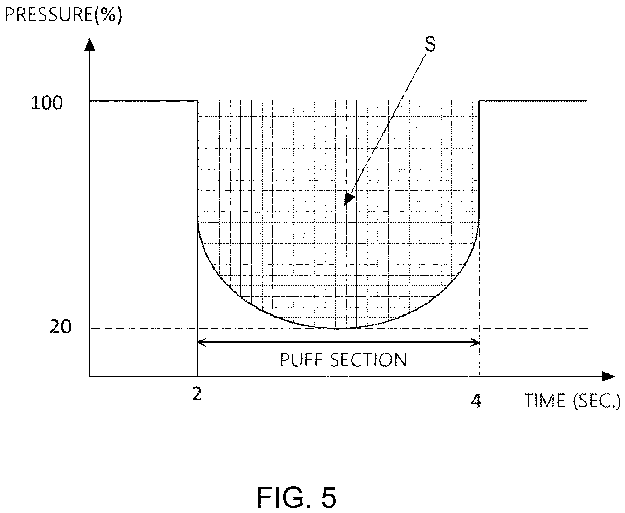

FIG. 5 is a graph illustrating a change in pressure over time detected by the puff factor detecting unit 400 when the puff factor detecting unit includes a pressure sensor. Due to the user's puff (inhalation) action, the pressure in the air flow path 130 temporarily decreases. FIG. 5 illustrates a case in which the pressure in the air flow path 130 in normal times, that is, when there is no puff, is calculated as 100% and the pressure decreases by up to 80% within a puff section of 2 to 4 seconds as a puff takes place. The controller 200 may integrate variations of pressure at this time with time to calculate a change in pressure area (an area of the portion indicated by S in FIG. 5) and estimate a puff flow rate based on the calculated change in pressure area. To this end, first, it may be necessary to derive a relationship between the integral value and the puff flow rate or a relationship between the integral value and the aerosol-forming substrate consumption amount by an experiment in advance and model or approximately model the relationship, or it may be necessary to acquire the puff flow rate or the consumption amount of the aerosol-forming substrate in a statistical manner, create a correspondence table, and store corresponding data in the controller 200 in advance. The controller 200 may derive a puff flow rate or a consumption amount of the aerosol-forming substrate corresponding to the calculated integral value by using the stored modeling information, which may be an approximation close to the actual consumption amount.

The controller 200 may calculate the consumption amount of the aerosol-forming substrate with the estimated puff flow rate and subtract the consumption amount of the aerosol-forming substrate from a previous residual amount of the currently inserted aerosol-forming article 1000 to calculate a current residual amount of the aerosol-forming substrate.

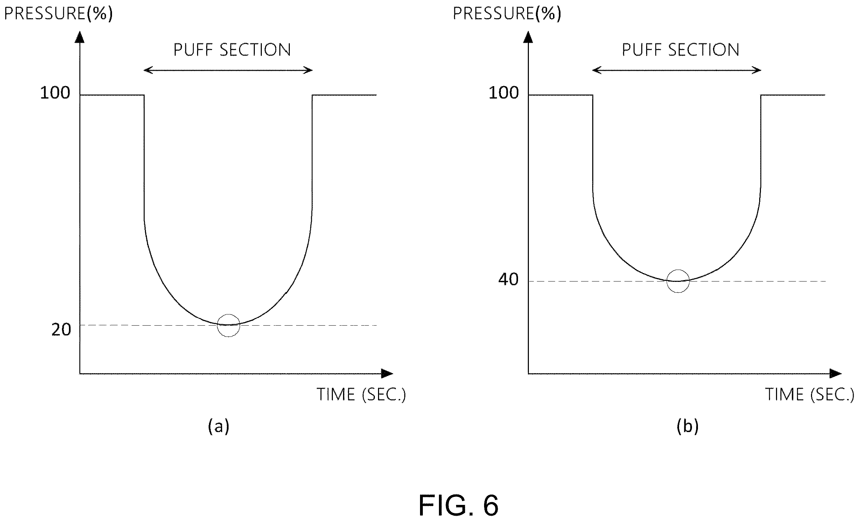

FIG. 6 is an exemplary graph illustrating a change in pressure over time detected by a pressure sensor of the puff factor detecting unit 400 in order to explain another embodiment of the controller 200. Referring to FIG. 6A, pressure in the air flow path 13 decreases by up to 80 % due to a puff action. Also, referring to FIG. 6B, pressure in the air flow path 130 decreases by up to 60 % due to a puff action.

In an embodiment, the controller 200 may estimate a puff flow rate based on the maximum value of the change in pressure over time detected by the puff factor detecting unit 400, that is, an extreme value. This is because the extreme value of the change in pressure may represent the integral value based on the fact that the aspect of the change in pressure over time due to the puff action, that is, the shape of the graph, is always similar. For example, the puff flow rate when the extreme value of the change in pressure is -80 % in the puff section as shown in FIG. 6A may be greater than the puff flow rate when the extreme value of the change in pressure is -60 % in the puff section as shown in FIG. 6B, and thus, by deriving a statistical correspondence table or relational modeling between the two through experiment in advance, the controller 200 may estimate the puff flow rate per puff and the consumption amount of aerosol-forming substrate through the puff factor detecting unit 400.

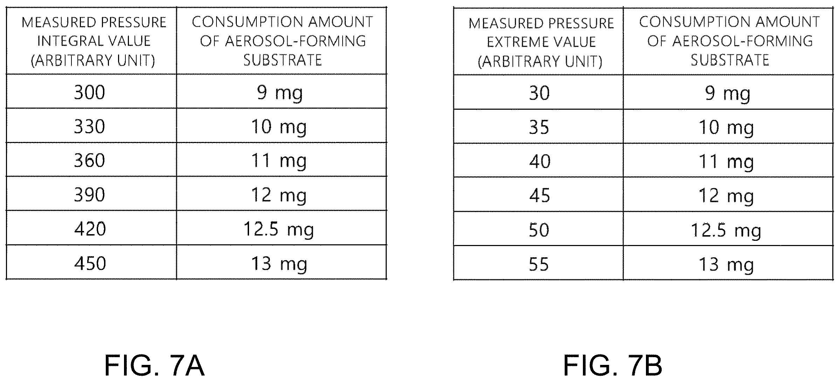

In another embodiment, the controller 200 may directly estimate a consumption amount of the aerosol-forming substrate per puff based on the integral value of the change in pressure over time or a peak value of the change in pressure over time, without estimating the puff flow rate. FIG. 7A is an example of a statistical correspondence table for the controller 200 to estimate a consumption amount of the aerosol-forming substrate based on the integral value of the change in pressure over time, which may be determined in advance by an experiment, and FIG. 7B is an example of a statistical correspondence table for the controller 200 to estimate a consumption amount of the aerosol-forming substrate based on an extreme value of the change in pressure over time. The controller 200 may store and hold the two tables converted into data in advance, and calculate a current residual amount by subtracting a corresponding consumption amount of the aerosol-forming substrate from a previous residual amount based on the measured integral value or extreme value.

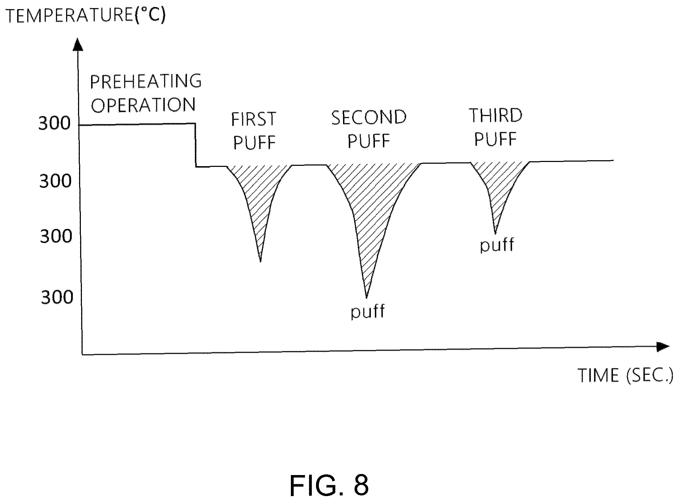

FIG. 8 is a graph of a change in temperature of the heating space 120 or the heating unit 300 over time detected by the puff factor detecting unit 400 when the puff factor detecting unit 400 includes a temperature sensor. In particular, the temperature graph of FIG. 8 represents temperature detection values during the execution of the aerosol generating function performed by the controller 200 using a basic temperature profile. The basic temperature profile may include, for example, a target temperature for a preheating operation and a target temperature after the preheating operation.

The temperature of the heating space 120 or the heating unit 300 may temporarily drop due to the user's puff (inhalation) action, and by detecting this, the controller 200 may estimate a consumption amount of the aerosol-forming substrate and calculate a residual amount of the aerosol-forming substrate. Referring to FIG. 8, the temperature of the heating space 120 or the heating unit 300 at normal times, that is, when there is no puff, maintains a temperature of about 250 °C, which is a target temperature after the preheating operation. However, since outside air may flow into the heating space 120 during the puff section and is discharged again together with the aerosol, the temperature may temporarily decrease and then returns to the target temperature of 250 °C due to a heating operation of the heating unit 300 under the control of the controller 200.

Based on the recognition that a variation of the temperature detection value relates to the puff flow rate or the consumption amount of the aerosol-forming substrate, the controller 200 may estimate the puff flow rate or the consumption amount of the aerosol-forming substrate in a similar manner as that of the case in which the puff factor detecting unit 400 includes the pressure sensor described above with reference to FIGS. 5 and 6. That is, the controller may calculate a temperature change area by integrating the variation in temperature over time as shown in FIG. 8 with time and estimate the puff flow rate based on the calculated temperature change area. Alternatively, the controller 200 may estimate the puff flow rate based on the maximum value of the change in temperature over time, that is, an extreme value. At this time, a statistical correspondence table or relational modeling between the integral value or extreme value of the change in temperature of the heating space 120 or the heating unit 300 over time that may be determined in the same manner as described above with reference to FIGS. 5 and 6, that is, for example, by an experiment in advance or stored in the controller 200 and the puff flow rate or the consumption amount of the aerosol-forming substrate may be used. The controller 200 may calculate the consumption amount of the aerosol-forming substrate with the estimated puff flow rate and subtract the consumption amount from the previous residual amount of the currently inserted aerosol-forming article 1000 to calculate the current residual amount of the aerosol-forming substrate.

When the factor related to the user's puff detected by the puff factor detecting unit 400 is whether the user's puff is generated, the controller 200 may approximately estimate the current residual amount of the aerosol-forming substrate by subtracting a certain consumption amount of the aerosol-forming substrate whenever the generation of the user's puff is detected. For example, the controller 200 may calculate the current residual amount of the aerosol-forming substrate by simply subtracting an average consumption amount of the aerosol-forming substrate per puff, which may be determined in advance by an experiment. Alternatively, the controller 200 may calculate an active consumption amount for each section by determining a rate at which the aerosol-forming substrate is actively consumed in the puff section, that is, a reference consumption rate, and multiplying the reference consumption rate by a duration of the corresponding section.

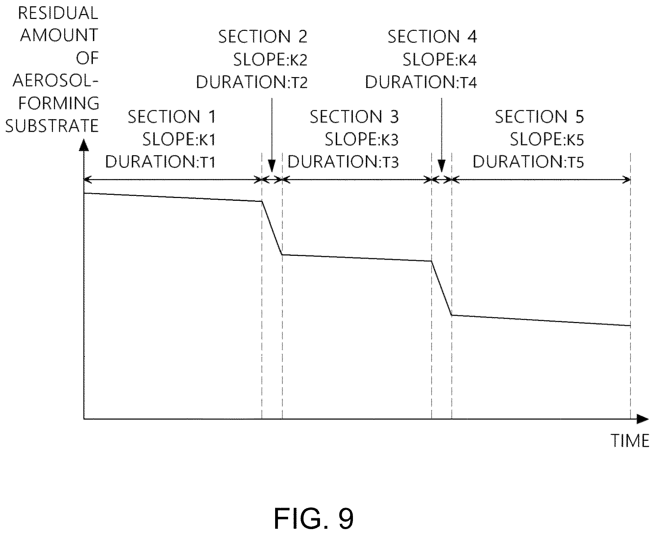

FIG. 9 is a graph of consumption of a residual amount of an aerosol-forming substrate residual amount consumption graph modeling how an aerosol-forming substrate in the aerosol-forming article 1000 is consumed in the aerosol generating device 1. Among the total sections of 1 to 5, sections 1, 3, and 5 correspond to pause sections between the user's puff actions. In addition, sections 2 and 4 are sections in which the user's puff takes place, indicating rapid consumption of the aerosol-forming substrate due to the puff action.

For example, in sections 2 and 4 representing the consumption amount during the user's puff action, in an embodiment, the reference consumption rate in the puff section remains constant and is equal to each other. That is, the slopes K2 and K4 maintain the same value (reference consumption rate). In another embodiment, respective reference consumption rates in the puff sections are constant and not the same as each other. In another embodiment, the reference consumption rates in the puff sections are not constant and are not the same as each other. However, since the puff section is relatively shorter than the pause section, the controller 200 may directly calculate the consumption amount of the aerosol-forming substrate after one puff as described above, in addition to the method of calculating the consumption amount by multiplying the reference consumption rate by the duration.

For example, the controller 200 may calculate the residual amount of the aerosol-forming substrate after the puff based on the residual amount of the aerosol-forming substrate before the puff. In this case, as the value of the residual amount of the aerosol-forming substrate before the puff increases, the reference consumption rate or consumption amount due to one puff may increase. Therefore, the controller 200 may store the currently calculated residual amount of the aerosol-forming substrate at the beginning of section 2, and when the section 2 ends, the controller 200 may calculate a residual amount by calculating the consumption amount of the aerosol-forming substrate consumed by puff in the section 2 by applying the reference consumption rate or the consumption amount of one puff corresponding to the stored residual value of the aerosol-forming substrate at the time of starting the section 2. The reference consumption rate for one puff or the consumption amount for one puff according to the residual amount of the aerosol-forming substrate may be determined in advance, for example, by an experiment, and the controller 200 may store and memorize the same.

Also, for example, the controller 200 may calculate the residual amount of the aerosol-forming substrate after the puff based on a temperature of the heating unit 300 before the puff. In this case, as the temperature of the heating unit 300 before the puff increases, the reference consumption rate or the consumption amount due to one puff may increase. Therefore, the controller 200 may store the current temperature of the heating unit 300 at the start of section 2, for example, obtained through a temperature sensor, and when the section 2 ends, the controller 200 may calculate a residual amount by calculating the consumption amount of the aerosol-forming substrate consumed by the puff of the section 2 by applying the reference consumption rate or the consumption amount of one puff corresponding to the stored current temperature value of the heating unit 300. The reference consumption rate of one puff or the consumption amount of one puff according to the current temperature of the heating unit 300 may be determined in advance, for example, by an experiment, and the controller 200 may store and memorize the same.

Also, for example, the controller 200 may calculate the residual amount of the aerosol-forming substrate after the puff based on a duration time of the puff, that is, duration T2 of the section 2 and duration T4 of the section 4. In this case, as the duration of the puff is longer, the consumption amount due to one puff may increase. Therefore, the controller 200 may calculate the residual amount by calculating the consumption amount of the aerosol-forming substrate consumed by the puff in the section 2 by applying the consumption amount per puff corresponding to the duration of the section 2, which may be obtained by the puff factor detecting unit 400, for example. The consumption amount of one puff according to the duration of the puff may be determined in advance by, for example, an experiment, and the controller 200 may store and memorize the same.

The slopes K1, K3, and K5 of the section 1, the section 3, and the section 5 each represent the consumption amount of the aerosol-forming substrate naturally consumed in the pause section, that is, a passive consumption amount. According to the simplest modeling, the slopes K1, K3, and K5 of the section 1, the section 3, and the section 5 are zero. That is, the passive consumption amount is not considered in modeling in the related art. However, actually, the heating unit 300 generates heat at a predetermined temperature even in the pause sections, and accordingly, the aerosol-forming substrate is gradually discharged as an aerosol and may be consumed.

According to an embodiment, the controller 200 may subtract the passive consumption amount represented by the slopes K1, K3, and K5 when calculating the residual amount of the aerosol-forming substrate for correction. That is, the controller 200 may calculate the passive consumption amount of each section by determining a rate at which the aerosol-forming substrate is passively consumed in the pause section, that is, the reference consumption rate, and multiplying the reference consumption rate by the duration of the corresponding section, and may subtract the passive consumption amount from the residual amount of the aerosol-forming substrate for correction.

In an embodiment, the reference consumption rates in the pause sections are maintained to be constant and are the same as each other. That is, the slopes K1, K3, and K5 maintain the same value (reference consumption rate). In another embodiment, respective reference rates in the pause sections are constant and not the same as each other. In another embodiment, the reference consumption rates in the pause sections are not constant and are not the same as each other. That is, the slopes K1, K3, and K5 of the pause sections, section 1, section 3, and section 5, may vary depending on other factors, and as these factors are considered, the residual amount of the aerosol-forming substrate calculated by the controller 200 may be more accurate.

For example, the rate at which the aerosol-forming substrate is passively consumed in the pause section, that is, the reference consumption rate, may be determined according to the residual amount of the aerosol-forming substrate. For example, as the value of the residual amount of the current aerosol-forming substrate increases, the reference consumption rate may further increase. Therefore, the controller 200 may store the currently calculated residual value of the aerosol-forming substrate at the beginning of section 1, and when the section 1 ends, the controller 200 may calculate the consumption amount of the aerosol-forming substrate consumed in the section 1 by multiplying the reference consumption rate corresponding to the residual value of the aerosol-forming substrate by the duration T1 of the section 1. In the present embodiment, since the residual amount of the aerosol-forming substrate decreases as the section continues, the magnitude comparison of the slope values of each section may be K1 > K3 > K5. The reference consumption rate according to the residual amount of the aerosol-forming substrate may be determined by, for example, an experiment in advance, and the controller 200 may store and memorize the same.

Also, for example, the reference consumption rate may be determined according to the temperature of the heating space 120 or the heating unit 300. For example, as the current temperature of the heating unit 300 increases, the reference consumption rate may increase. Therefore, the controller 200 may store the current temperature of the heating space 120 or the heating unit 300 at the start of section 1, for example, obtained through the temperature sensor, and when the section 1 ends, the controller 200 may calculate the consumption amount of the aerosol-forming substrate consumed in the section 1 by multiplying the reference consumption rate corresponding to the stored current temperature value of the heating space 120 or the heating unit 300 by the actual duration T1 of the section 1. Alternatively, in another embodiment, the passive consumption amount may be calculated more dynamically by measuring the temperature of the heating unit 300 several times even in the section 1 and applying a different reference consumption rate to each time. The reference consumption rate according to the current temperature of the heating unit 300 may be determined by an experiment in advance, for example, and the controller 200 may store and memorize the same.

Also, for example, the reference consumption rate may be determined according to an elapsed time of the current pause section. This means that the reference consumption rate may be dynamically changed even within one section. In addition, this means that the reference consumption rate may be changed to a predetermined acceleration value in one section. This reference is meaningful because, for example, it can be assumed that the current temperature of the heating unit 300 and the current residual amount of the aerosol-forming substrate will change at a relatively constant level according to the elapsed time of the pause section. Therefore, at the start of section 1, the controller 200 may apply an acceleration value (a change level of K1) that may be determined in advance by, for example, an experiment, and when section 1 finally ends, the controller 200 may calculate and correct a consumption amount of the aerosol-forming substrate consumed in the section 1. Alternatively, the controller 200 may store a reference consumption rate table for each elapsed time of the pause section, which is determined by an experiment in advance, and calculate and correct a consumption amount of the aerosol-forming substrate consumed in the section 1 by applying the reference consumption rate determined for each elapsed time.

The controller 200 may estimate the consumption amount of the aerosol-forming substrate during one puff by the above methods, and thus, the controller 200 may continuously calculate the current residual amount by subtracting the consumption amount due to one puff from the previous residual amount of the aerosol-forming substrate. In order to calculate the residual amount by the controller 200, an initial amount of the aerosol-forming substrate of the aerosol-forming article may be input to the controller 200 in advance.

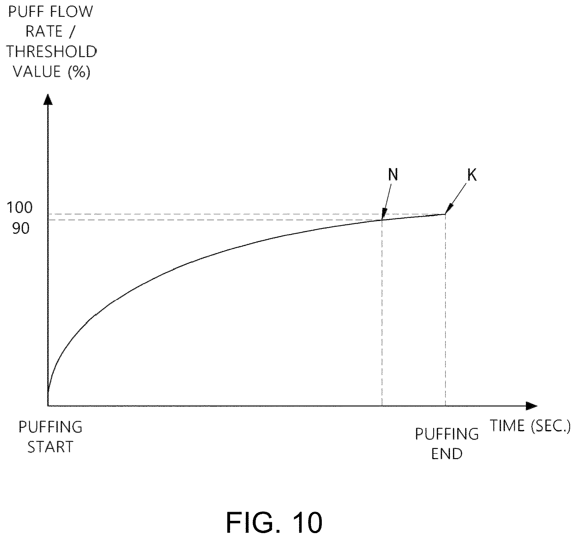

FIG. 10 is a graph illustrating the consumption amount of an aerosol-forming substrate or a flow rate of a puff during one puff, estimated by the controller 200, over time according to an embodiment of the present disclosure. The controller 200 may measure or estimate the flow rate of a puff in real time according to the detected contents of the puff factor detecting unit 400, and accordingly, the controller 200 calculates the consumption amount of the aerosol-forming substrate in real time when the user performs a puff action. The controller 200 may represent the ratio of the consumption amount of the aerosol-forming substrate or the puff flow rate calculated in real time with respect to the consumption amount of the aerosol-forming substrate or puff flow rate during one puff, which may be arbitrarily determined, as a percentage. When the consumption amount of the aerosol-forming substrate calculated by the controller 200 in real time reaches 100% as indicated by point K of FIG. 10, that is, when a predetermined stop threshold value is reached, the controller 200 may control the heating unit 300 to stop heating. Through this control, the user may inhale a relatively constant amount of the aerosol-forming substrate for each puff, so that uniformity of taste and mist amount may be guaranteed. In addition, a case in which the heating unit 300 is unnecessarily heated for a longer period of time even though a sufficient amount of the aerosol is generated and inhaled is reduced, thereby reducing power consumption at the time of using the aerosol generating device 1 to lengthen a usage time after charging the battery 700 once.

According to an embodiment, the controller 200 may notify the user in advance that the aerosol is sufficiently inhaled or the heating unit 300 stops heating before the consumption amount of the aerosol-forming substrate or the flow rate of the puff calculated in real time reaches the stop threshold value. That is, when the puff flow rate measured or estimated by the puff factor detecting unit 400 reaches a predetermined warning threshold value, for example, 90% of the stop threshold value, the controller 200 may notify the user about heating stop through the notification unit 500.

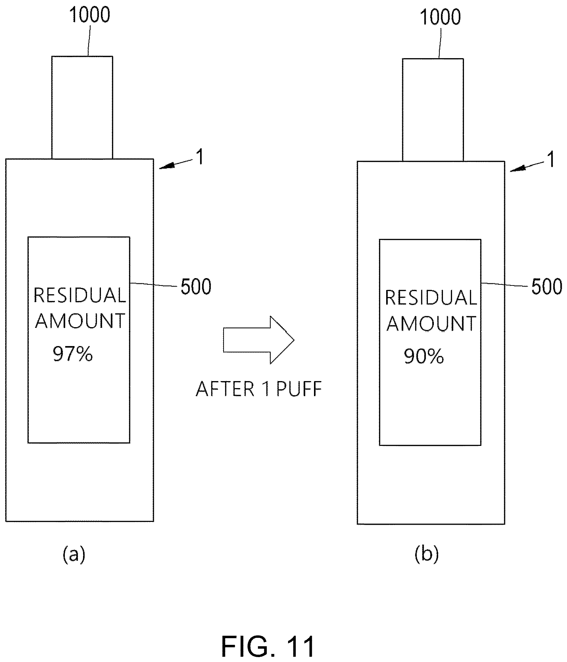

The notification unit 500 may be, for example, a display, a haptic actuator, or a microspeaker. The notification unit 500 generates at least one of a haptic effect, a visual effect, or a sound effect under the control of the controller 200 to inform the user that the aerosol has been sufficiently inhaled or that the heating unit 300 stops heating in advance. In addition, the controller 200 may inform the user of the calculated residual amount of the aerosol-forming substrate through the notification unit 500 in real time. For example, as shown in (a) and (b) of FIG. 11, the notification unit 500 may display the calculated residual amount of the aerosol-forming substrate through the display or inform the user through vibrations or sound each time a predetermined value is reached.

Also, according to an embodiment, the controller 200 may calculate the number of possible puffs of the mounted aerosol-forming article 1000 based on the calculated residual amount of the aerosol-forming substrate. For example, the controller 200 may predict the residual number of puffs of the mounted aerosol-forming article 1000 according to a predetermined rule based on the currently calculated residual amount and the consumption amount of the aerosol-forming substrate according to one puff of the user that is previously measured. For example, the controller 200 may predict the number of remaining puffs based on the previously measured average value of the consumption amount of the aerosol-forming substrate according to one puff of the user. In addition, the controller 200 may inform the user of the number of puffs of the mounted aerosol-forming article through the notification unit 500 according to an embodiment. Accordingly, the user may predict when the currently mounted aerosol-forming article 1000 will be used up, and may predict when the operation of the device will end and when the aerosol inhalation will stop.

According to an embodiment, when the aerosol generating device 1 is stopped during use, the controller 200 may store the residual amount of the aerosol-forming substrate calculated up to that time, so that when the user restarts the aerosol generating device 1 with the same aerosol-forming article 1000 thereafter, the controller 200 may load the stored residual amount of the aerosol-forming substrate to continue to calculate the residual amount of the aerosol-forming substrate.

For example, the aerosol generating device 1 may include a cigarette insertion detecting unit (not shown) capable of detecting insertion and withdrawal of the cigarette-type aerosol-forming article 1000 on one side, particularly, on one side of the heating space 120. The cigarette insertion detecting unit may be an optical sensor, a proximity sensor, or a contact sensor and may detect insertion of the aerosol-forming article 1000 into the heating space 120 or insertion and withdrawal of the aerosol-forming article 1000 into and from the heating space 120.

When the aerosol-forming article 1000 is detected through the cigarette insertion detecting unit in calculating the residual amount of the aerosol-forming substrate, the controller 200 may load the initial amount of the stored aerosol-forming substrate and calculate a residual amount of the aerosol-forming substrate by subtracting the consumption amount of the aerosol-forming substrate according to one puff, which is estimated through the detection contents of the puff factor detecting unit 400 thereafter. When the withdrawal and insertion of the aerosol-forming article 1000 are detected through the cigarette insertion detecting unit, that is, when the replacement of the aerosol-forming article 1000 is detected, the controller 200 may initialize the calculated residual amount of the aerosol-forming substrate. That is, the initial amount of the aerosol-forming substrate stored may be loaded again. Meanwhile, if the replacement of the aerosol-forming article 1000 is not detected and the aerosol generating device 1 is stopped during use, the residual amount of the aerosol-forming substrate calculated until then is stored. Accordingly, when the aerosol generating device 1 is restarted, the controller 200 may continue to calculate the residual amount of the aerosol-forming substrate by loading the stored residual amount of the aerosol-forming substrate.

According to an embodiment, the controller 200 may determine whether the puff flow rate measured or estimated by the puff factor detecting unit 400 falls within an abnormal value range. The controller 200 stores, for example, a normal value range of puff flow rate determined in advance through an experiment and an abnormal value range of the puff flow rate other than the normal value range, so that the puff flow rate measured or estimated by the puff factor detecting unit 400 is within the normal value range or the abnormal value range. Alternatively, for example, the controller 200 may determine a normal value range of the puff flow rate and an abnormal value range, which is a range other than the normal value range, based on an average value of the puff flow rate measured or estimated over a predetermined use cycle of the aerosol generating device 1.

When the puff flow rate measured or estimated by the puff factor detecting unit 400 falls within the abnormal value range, the controller 200 determines that the air flow path 130 is clogged due to contamination or inflow of foreign substances. Accordingly, in this case, the controller 200 may control the aerosol generating device 1 to operate in a cleaning mode. The cleaning mode may be, for example, a mode informing the user that cleaning is required through the notification unit 500. Alternatively, for example, the cleaning mode may be a mode of operating a cleaning device (not shown) that may be included in the aerosol generating device 1, such as an air jet nozzle.

According to an embodiment, the controller 200 may store a basic temperature profile for performing an aerosol generating function for one aerosol-forming article. The basic temperature profile may include a target temperature according to time or a target temperature according to puffs. In addition, the controller 200 may variably control the target temperature of the basic temperature profile according to factors related to the user's puff or the calculated residual amount of the aerosol-forming substrate, and store a variable temperature profile by matching the varied target temperature to each puff. To this end, the aerosol generating device 1 may further include a temperature sensor 800 that detects a temperature of the heating space 120 or the heating unit 300 and applies the detected temperature value to the controller according to an embodiment. However, if the puff factor detecting unit 400 includes a temperature sensor that detects the temperature of the heating space 120 or the heating unit 300, the temperature sensor 800 may be replaced with the temperature sensor included in the puff factor detecting unit 400.

In the variable temperature profile, for example, a target temperature of a first puff and a preheating operation is 300 °C, a target temperature of a third puff is 258 °C, and a target temperature of a tenth puff is 252 °C. In addition, the controller 200 may store the highest temperature value of the target temperature of the basic temperature profile and the variable temperature profile, and change the highest temperature value according to a factor related to the user's puff or the calculated residual amount of the aerosol-forming substrate. This process is also described in detail below.

The controller 200 performs a heating operation by applying power applied from the battery 700 to the heating unit 300 according to the target temperature and the temperature detection value included in the basic temperature profile or the variable temperature profile, and performs an aerosol generating function.

The controller 200 stores a predetermined reference residual amount with respect to the residual amount of the aerosol-forming substrate. The controller 200 may calculate the residual amount of the aerosol-forming substrate as described in detail above. At this time, the controller 200 may compare the calculated residual amount of the aerosol-forming substrate with the reference residual amount during the current puff or at the end of the current puff, and if the calculated residual amount is equal to or greater than the reference residual amount, the controller 200 may vary the target temperature of the basic temperature profile in a next puff. If the calculated residual amount is equal to or greater than the reference residual amount, the controller 200 changes the target temperature of the basic temperature profile in a next puff to decrease by a predetermined amount. Even if the target temperature is lowered in this way, when the residual amount of the aerosol-forming substrate is equal to or greater than the reference residual amount, the puff flow rate, which is a discharge amount of the aerosol, is sufficient. However, if the calculated residual amount is less than the reference residual amount, the controller 200 changes the target temperature of the basic temperature profile to increase by a predetermined amount in the next puff or from the next puff. The controller 200 may include and store the changed target temperature in the variable temperature profile, and control the heating unit 300 according to a temperature detection value from the temperature sensor 800 or the temperature sensor included in the puff factor detecting unit 400 such that the temperature detection value reaches the changed target temperature in the next puff. In addition, the controller 200 maintains the target temperature below a predetermined highest temperature value even though the target temperature is varied.

In addition, the controller 200 may change a target temperature of a next puff based on a change in pressure in the air flow path 130 or a flow rate of air flowing through the air flow path 130 detected by the puff factor detecting unit 400 or a change in temperature of the heating space 120 or the heating unit 300. For example, the target temperature of the next puff may be varied based on the puff flow rate calculated by the controller 200 or the consumption amount of aerosol-forming substrates based on the detection values detected by the puff factor detecting unit 400.

After the end of the current puff, the controller 200 may increase a target temperature of a next puff by a preset size if the calculated puff flow rate or the consumption amount of the aerosol-forming substrate is smaller than an average puff flow rate or a certain reference puff flow rate, and may decrease the target temperature of the next puff by a preset size if the calculated puff flow rate or the consumption amount of the aerosol-forming substrate is greater than the average puff flow rate or the certain reference puff flow rate.

By varying the target temperature, even in the last puff of a reference number of puffs, aerosol may be discharged at an average puff flow rate or a flow rate close to a predetermined reference puff flow rate. That is, even if the puff time or the user's smoking method is different, there is an effect of making the puff flow rate or the consumption amount of the aerosol-forming substrate uniform in each puff.

In addition, the controller 200 acquires a variable input for the reference number of puffs from the user through the input unit 600. The reference number of puffs is the number of times selected in consideration of the reference amount of discharge or the reference puff flow rate at which the aerosol-forming substrate needs to be discharged. Therefore, if the variable input is a decrease input, that is, if the reference number of puffs is reduced, the total puff flow rate of the aerosol-forming substrate may also be reduced. Thus, when the reference number of puffs is reduced, the controller may increase the puff flow rate in each puff, and increase the target temperature of each puff by a set size in consideration of the reduced number of puffs. Due to the increase in temperature, the average target temperature of the puff is also increased. Conversely, if the variable input is an increase input, that is, if the reference number of puffs is increased, the controller 200 may lower the target temperature of each puff by a preset size in consideration of the increased number of puffs, so that a sufficient amount of mist may be discharged even in the last puff. Due to this temperature decrease, the average target temperature of the puff also decreases. By varying the target temperature, the total puff flow rate of the aerosol-forming substrate discharged from a single aerosol-forming article is maintained constant.

In addition, the controller 200 may continuously operate the heating operation of the heating unit 300 when the aforementioned total amount of mist is less than or equal to a reference discharge amount or reference puff flow rate of the aerosol-forming substrate described above, that is, when there is a residual amount of the aerosol-forming substrate.

According to various embodiments, at least a portion of the device (e.g., modules or functions thereof) or the method (e.g., operations) may be implemented, for example, as instructions stored in a non-transitory computer-readable storage medium in a programming module form. When the instructions are executed by a processor, the processor may execute a function corresponding to the instructions. The computer-readable storage medium may be, for example, the memory.

The non-transitory computer-readable recording medium may include magnetic media, such as a hard disk, a floppy disk, and a magnetic tape, optical media, such as a compact disc read only memory (CD-ROM) and a digital versatile disc (DVD), magneto-optical media, such as a floptical disk, and hardware devices (e.g., read only memory (ROM), random access memory (RAM), or flash memory). In addition, program instructions may include high class language codes, which may be executed in a computer by using an interpreter, as well as machine codes made by a compiler. The hardware devices described above may be configured to operate as one or more software modules to perform the operations of various embodiments, and vice versa.

A module or programming module according to various embodiments may include or exclude at least one of the above-discussed components or further include any other component. The operations performed by the module, programming module, or any other component according to various embodiments may be executed sequentially, in parallel, repeatedly, or by a heuristic method. Additionally, some operations may be executed in different orders or omitted, or any other operation may be added. While the present disclosure has been shown and described in connection with the embodiments, it will be apparent to those skilled in the art that modifications and variations may be made without departing from the spirit and scope of the invention as defined by the appended claims.

## Claims

1. An aerosol generating device comprising:
a case including a heating space and an air flow path connecting the heating space to an external space;
a heating unit heating an aerosol-forming article inserted into the heating space;
a puff factor detecting unit detecting a factor related to a user's puff; and
a controller controlling at least the heating unit and calculating a residual amount of the aerosol-forming substrate based on detection contents of the puff factor detecting unit,
wherein the factor related to the user's puff detected by the puff factor detecting unit includes any one or more of a change in pressure in the air flow path, a flow rate of air flowing through the air flow path, a change in temperature in the heating space or the heating unit, or whether a user's puff is generated.

2. The aerosol generating device of claim 1, wherein the puff factor detecting unit detects at least any one of the change in pressure in the air flow path, the flow rate of air flowing through the air flow path, the change in temperature of the heating space or the heating unit, or whether the user's puff is generated, and the controller calculates the residual amount of the aerosol-forming substrate based on any one of the change in pressure in the air flow path over time, the flow rate of air flowing through the air flow path, the change in temperature of the heating space or the heating unit over time, or whether the user's puff is generated, detected by the puff factor detecting unit.

3. The aerosol generating device of claim 2, wherein the puff factor detecting unit detects at least the change in pressure in the air flow path, and the controller calculates the residual amount of the aerosol-forming substrate based on an integral value or an extreme value of the change in pressure in the air flow path over time detected by the puff factor detecting unit.

4. The aerosol generating device of claim 2, wherein the puff factor detecting unit detects at least whether the user's puff is generated, and the controller calculates a residual amount of the aerosol-forming substrate after the puff based on any one of a residual amount of the aerosol-forming substrate before the puff, a temperature of the heating space or the heating unit before the puff, or a duration of the puff.

5. The aerosol generating device of claim 1, wherein, when calculating the residual amount of the aerosol-forming substrate, the controller corrects the residual amount of the aerosol-forming substrate by subtracting a consumption amount (a passive consumption amount) of the aerosol-forming substrate naturally consumed in a pause section between the user's puff actions.

6. The aerosol generating device of claim 5, wherein the controller calculates the passive consumption amount by multiplying a reference consumption rate by a duration of the pause section.

7. The aerosol generating device of claim 6, wherein the reference consumption rate is determined based on any one of the residual amount of the aerosol-forming substrate, the temperature of the heating space or the heating unit, or an elapsed time of a current pause section.

8. The aerosol generating device of claim 2, wherein the puff factor detecting unit detects at least the change in temperature of the heating space or the heating unit, and the controller calculates the residual amount of the aerosol-forming substrate based on an integral value or an extreme value of the change in temperature of the heating space or the heating unit over time, detected by the puff factor detecting unit.

9. The aerosol generating device of claim 1, further comprising:
a notification unit generating at least one of a haptic effect, a visual effect, or a sound effect for user notification,
wherein the controller calculates the number of possible puffs of the mounted aerosol-forming article based on the calculated residual amount of the aerosol-forming substrate, and informs the user of the calculated number of possible puffs through the notification unit.

10. The aerosol generating device of claim 2, wherein the puff factor detecting unit detects at least the flow rate of air flowing through the air flow path, and the controller stops the heating of the heating unit when the flow rate of the air flowing through the air flow path detected by the puff factor detecting unit reaches a predetermined stop threshold value.

11. The aerosol generating device of claim 1, wherein, when the operation of the aerosol generating device is stopped during use, the controller stores a residual amount of the aerosol-forming substrate calculated by then, and when the aerosol generating device is restarted, the controller loads the stored residual amount of the aerosol-forming substrate and continue to calculate the residual amount of the aerosol-forming substrate.

12. The aerosol generating device of claim 1, wherein the controller initializes the calculated residual amount of the aerosol-forming substrate when the inserted aerosol-forming article is replaced.

13. The aerosol generating device of claim 1, further comprising:
a temperature sensor detecting a temperature of the heating space or the heating unit and applying a temperature detection value to the controller,
wherein the controller controls the heating unit according to a temperature profile including a target temperature and the temperature detection value to heat the aerosol-forming substrate of the aerosol-forming article to generate an aerosol, and adjusts a target temperature of a next puff by reflecting the factor related to the user's puff detected by the puff factor detecting unit or the calculated residual amount of the aerosol-forming substrate.

14. The aerosol generating device of claim 13, wherein the controller decreases a target temperature when the calculated residual amount of the aerosol-forming substrate is equal to or greater than a reference residual amount, and increases the target temperature when the calculated residual amount of the aerosol-forming substrate is less than the reference residual amount.

15. The aerosol generating device of claim 1, further comprising:
a temperature sensor detecting a temperature of the heating space or the heating unit and applying a temperature detection value to the controller; and
an input unit acquiring a variable input for a basic number of puffs from the user and applying the acquired variable input to the controller,
wherein the controller increases a target temperature when the basic number of puffs is decreased, and decreases the target temperature when the basic number of puffs is increased.
